(19) Europäisches Patentamt
European Patent Office
Office européen des brevets

(11) EP 1 215 615 A2

(12) EUROPÄISCHE PATENTANMELDUNG

(43) Veröffentlichungstag:
**19.06.2002 Patentblatt 2002/25**

(51) Int Cl.$^7$: **G06F 19/00**

(21) Anmeldenummer: **01129193.7**

(22) Anmeldetag: **10.12.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **18.12.2000 DE 10063052**

(71) Anmelder: **PE Diagnostik GmbH**
**04416 Markkleeberg (DE)**

(72) Erfinder: **Bitterlich, Norman**
**09114 Chemnitz (DE)**

(74) Vertreter: **Seerig & Hübner**
**Patentanwälte,**
**Postfach 213**
**09026 Chemnitz (DE)**

# (54) Gen-und risikoassoziierte Tumormarkerauswahl

(57) Aufgabe der Erfindung ist es, eine gen- und risikoassoziierte Tumormarkerauswahl zu entwickeln, bei der kostengünstig das Screening-Verfahren anwendbar ist.

Erfindungsgemäß wird die Aufgabe durch folgende Schritte gelöst:

1. ein Gen-Test wird aus dem Zellgewebe einer Person hinsichtlich krebsrelevanter Gen-Mutationen entnommen,
2. Krebsrisikofaktoren werden dem jeweiligen Karzinom zugeordnet,
3. aus der Gleichung

$$K(k) = 1-\left(\prod_{m=1}^{m_M}(1-M(m)\cdot ZM(m,k))\right)\cdot\left(\prod_{r=1}^{r_R}(1-R(r)\cdot ZR(r,k))\right)$$

wird der organspezifischen Karzinomverdacht K(k) ermittelt,
4. ein Markerprofil für die Parameteranalyse wird ausgewählt,
5. Vornahme einer Markerprofilauswertung,
6. anhand der ermittelten Markerwerte werden Aussagen zur Diagnostik mittels bekannter fuzzy-basierter Auswerteverfahren vorgenommen,
7. die Bewertungsergebnisse werden gewichtet, um zu entscheiden, ob ein Diagnoseverfahren zu empfehlen ist, oder ob eine Wiederholungsmessung durchzuführen ist, oder ob eine neue Bewertung durchzuführen ist.

EP 1 215 615 A2

## Beschreibung

Die Erfindung betrifft eine gen- und risikoassoziierte Tumormarkerauswahl.

Mit Gen-Tests können heute verschiedene Gen-Mutationen nachgewiesen werden, deren Auftreten statistisch nachweislich ein erhöhtes Risiko für Tumorerkrankungen bedeuten. Dabei sind die Gen-Informationen sehr spezifisch auf Körperorgane ausgerichtet. In zahlreichen Veröffentlichungen werden diese Zusammenhänge ausführlich beschrieben (vgl. Dong, J. et al.: A high proportion of mutations in the BRCA1 gene in German breast/ovarian cancer families with clustering of mutations in the 3'third of the gene". In: Human Genetics Vol. 103, 2 (1998), pp 154-161; Pitterle, D.M. et al. "Lung cancer and the human gene for ribonucleotide reductase subunit M1 (RRM1)." In: Mammalian Genome 10 (1999), pp 916-922; Matias-Guiu, X.; Prat, J.: "Molecular pathology of ovarian carcinomas". In: Virchows Archiv Vol 433, 2 (1998) pp 103-111; Saran, K.K. et al.: "Genetics of bladder cancer". In: Journal of Molecular Medicine Vol. 74, 8 (1996), pp 441-445. U.v.a. mehr). Verfahren zur Erkennung von krebsrelevanten Mutationen sind veröffentlicht (vgl. "Detection method for C-RAF-1 genes" (WO93/06248), "Prostate cancer gene" (WO99/32644), "Method for detecting pre-cancerous cells using P90 antibodies or probes" (WO94/00601), "Functional assay for tumor suppressor genes" (WO94/08049) u.v.a. mehr).

Ein Gen-Test bezüglich des Krebsrisikos ist insbesondere dann angeraten, wenn in der Familie gehäuft Tumorerkrankungen feststellbar waren, so dass der begründete Verdacht einer vererblichen Veranlagung zur Tumorbildung gegeben ist. Für die Gen-Information ist (im Rahmen der Testgenauigkeit) ein einmaliger Test ausreichend. Ein positives Testergebnis zeigt ein erhöhtes Risiko an, ein negatives Testergebnis schließt vererbtes erhöhtes Risiko aus. Ein negatives Testergebnis entbindet damit nicht der allgemein üblichen Aufmerksamkeit zur Krebs-(früh) Erkennung. Ein positives Testergebnis führt aber darüber hinaus dringend zu der Empfehlung, den bekannten Methoden der Krebsfrüherkennung besondere Aufmerksamkeit zu widmen. Es sind die modernen Möglichkeiten als Screening-Verfahren gezielt, effizient und regelmäßig anzuwenden. Die bekannten Untersuchungsmethoden, wie beispielsweise Bronchoskopie, erweisen sich dafür nicht nur wegen der Kosten als ungeeignet. Sie stellen insbesondere eine hohe Patientenbelastung dar, die einer regelmäßigen Routine-Überwachung im symptomfreien Zustand entgegenstehen. Damit wird die bereits hohe psychische Belastung aus dem Wissen um ein erhöhtes Risiko noch verstärkt. Tumormarkeranalysen können hier einen Beitrag leisten (vgl. Lamerz, R.; Stieber, P.: "Tumormarker", In: Der Onkologe Vol. 4, 11 (1998), pp 1067-1976). Prinzipiell können Tumormarker, also solche Stoffe, die als Indikatoren für malignes Zellwachstum interpretiert werden können, einen solchen Verlauf veranschaulichen, da deren quantitativen Labormesswerte in hohem Maße mit dem Fortschritt einer Tumorausprägung korrellieren. Mulitparameteranalysen ermöglichen mit modernen Auswertemethoden eine Leistungssteigerung für den Tumormarkereinsatz (vgl. Keller, T. et al.: "Tumour markers in the diagnosis of bronchial carcinoma: new options using fuzzy logic based tumour marker profiles." In: J Cancer Res Clin Oncol Vol. 124 (1998) pp 565-574). Obwohl Tumormarker wegen ihrer geringen Sensitivität und Spezifität den Anforderungen allgemeiner Screening-Verfahren nicht entsprechen, werden sie bereits erfolgreich für die Verlaufskontrolle oder bei Risikofaktoren eingesetzt. Gen-Test-Ergebnisse zählen bislang noch nicht zum screening-induzierenden Faktoren.

Bis heute sind mehr als 30 Tumormarker im Blut bekannt. Man unterscheidet Marker 1. und 2. Wahl in Abhängigkeit ihres Beitrages zur Krebserkennung. Aus den Ergebnissen der Markeranalyse lässt sich zwar die Lokalisation des Primärtumors abschätzen (vgl. Pecen, L. et al.: "methodology of fixing the possible localization of the unknown primary tumor based on patient sex, age and selectet tumor markers", In: Journal Tumor Marker Oncology Vol. 11, 2 (1996) pp 110-119), dies setzt aber voraus, dass ein breites Markerprofil analysiert wird. Die damit verbundenen Kosten stehen einer solchen Vorgehensweise entgegen.

Aufgabe der Erfindung ist es, eine gen- und risikoassoziierte Tumormarkerauswahl zu entwickeln, bei der kostengünstig das Screening-Verfahren anwendbar ist.

Erfindungsgemäß wird die Aufgabe entsprechend Anspruch 1 gelöst.

Nachfolgend wird die Erfindung anhand eines Ausführungsbeispieles näher erläutert. Die dazugehörige Zeichnung zeigt einen Ablaufplan einer gen- und risikoassoziierte Tumormarkerauswahl.

1. Eine Person A unterzieht sich einem Gen-Test hinsichtlich krebsrelevanter Gen-Mutationen M(1)..., $M(m_M)$. Das Ergebnis der Untersuchungen wird in Form einer elektronisch lesbaren tabellarischen Übersicht ausgegeben, in der die gesuchten Mutanten und das Suchergebnis: Mutation nicht beobachtet = 0; Mutation beobachtet = 1 zusammengestellt werden:

| | |
|---|---|
| RRM1 | 1 |
| BRCA1 | 0 |
| BRCA2 | 0 |
| ... | |

2. Ein Fragebogen, der zur Erfassung von Krebsrisikofaktoren R(1) ... $R(r_R)$ entsprechend dem öffentlichen Wissensstand erstellt ist, wird durch Befragung der Person als karzinomspezifische Einflussfaktoren abgeglichen und dem jeweiligen Karzinom zugeordnet. So sind Einflussfaktoren bekannt, wie:

| | |
|---|---|
| Tabakkonsum | Zigarettenzahl (ZE) pro Tag |
| Alkoholkonsum | Trinkeinheiten (TE) pro Tag |
| UV-Strahlung | Intensität (Anzahl der Tage im Jahr mit mehr als einer Stunde intensiver Belastung) (I) |

Aus der Befragung der Person ergeben sich für die Einflussfaktoren folgende Angaben:

$$ZE = 20,\ TE = 3,\ I = \frac{20\,Std}{365\,Tage} = 0{,}06$$

3. Die Aussagen der Verfahrensschritte 1. und 2. werden mittels einer tabellarischen Übersicht über den Zusammenhang zwischen organspezifischen Karzinomen und Gen-Mutationen sowie organspezifischen Karzinomen und den Krebsrisikofaktoren auf ihren potentiellen Risikoanteil zahlenmäßig dargestellt, wobei die Tabellenwerte grundsätzlich mit dem Maximalwert 1 zu begrenzen sind.

| Mutanten Karzinome | RRM1 | BRCA1 | ... | | Tabak | Alkohol | UV-Strahlung | ... |
|---|---|---|---|---|---|---|---|---|
| Lunge | 1 | 0 | | | 0,10*ZE/Tag (1,0) | 0 | 0 | |
| Brust | 0 | 0,7 | | | 0,02 *ZE/Tag (0,4) | 0,05*TE/Tag (0,15) | 0 | |
| Haut | 0 | 0 | | | 0 | 0,01*TE/Tag (0,03) | 1*\| (0,06) | |
| ... | | | ZM(m,k) | | | | | ZR(r,k) |

Beispiel der Zusammenhänge zwischen Karzinomen und Mutanten/Risikofaktoren (in Klammern:konsumabhängige Personendaten).

Die Zahlenangaben widerspiegeln die Stärke des Zusammenhanges (Wert 0 ... kein Zusammenhang bekannt/ 1 ... Zusammenhang erwiesen). Die Wertangabe ist eine Interpretation des Wissensstandes.

4. Nachfolgend wird die zahlenmäßige Darstellung des Untersuchungsergebnisses bezüglich der Mutation m mit M(m) und die zahlenmäßige Darstellung des Einflusses des Krebsrisikofaktors r mit R(r) bezeichnet, um aus der Gleichung

$$K(k) = 1-\left(\prod_{m=1}^{m_M}(1-M(m)\cdot ZM(m,k))\right)\cdot\left(\prod_{r=1}^{r_R}(1-R(r)\cdot ZR(r,k))\right)$$

den organspezifischen Karzinomverdacht K(k) bezüglich des Karzinoms K zu ermitteln. Der Karzinomverdacht K (k) ist um so größer, je mehr Verdachtsmomente zum Gesamtwert beitragen. Tabelleneintragungen 0 bzw. fehlende Angaben zu Mutanten oder Risikofaktoren beeinflussen den Karzinomverdacht K(k) nicht. Für die Beispieldaten ergeben sich folgende Werte:

K(Lunge) = 1,00
K(Brust) = 0,49
K(Haut) = 0,01

5. Aus der Fachliteratur sind organspezifische Marker 1. und 2. Wahl bekannt. Für das Beispiel sind folgende

organspezifische Marker angegeben:

| Marker Organ | NSE | CEA | CYFRA | CA19-9 | ... |
|---|---|---|---|---|---|
| Lunge | 1 | 0 | 0,5 | - | |
| Brust | 0 | - | 1 | 0,5 | |
| Haut | - | - | - | - | A(k,m) |

Organspezifische Marker 1. Wahl (1) und 2. Wahl (0,5) unspezifisch (0) bzw. relevant (-).

Die einzelne Wichtung W(m) der Marker m in Verbindung mit dem organspezifischen Karzinomverdacht k(k) wird für jeden einzelnen Marker m aus der Gleichung

$$W(m) = \min \left\{ 1; \sum_{m=1, A(k,m)="-"}^{m_W} K(k) \cdot A(k,m) \right\}$$

ermittelt werden, wobei A(k,m) die Eignung des Markers m als Indikator für Karzinome K am Organ einen Zahlenwert zwischen 0 und 1 ausweist. Für die Beispieldaten findet man

W(NSE) = 1,00
W(CEA) = 0,00
W(CYFRA) = 0,99
W(CA19-9) = 0,245

Auf der Basis dieser Ergebnisse wird das Markerprofil ausgewählt: NSE-CYFRA-CA19-9

6. Eine Probennahme und Labormessung für die Markerprofilauswertung erfolgt nach den bekannten Arbeitsschritten zur Bestimmung der Markerwerte in Blutserum. Für das Ausführungsbeispiel sind folgende Markerwerte L(m) gemessen:

L(NSE) = 12,50 Units
L(CYFRA) = 2,74 Units
L(CA19-9) = 7,98 Units

7. Anhand der gemessenen Markerwerte L(m) werden Aussagen zur Karzinomdiagnostik mit Hilfe bekannter fuzzybasierter Auswerteverfahren (vgl. Keller, T. et al.: "Tumour markers in the diagnosis of bronchial carcinoma: new options using fuzzy logic based tumour marker profiles." In: J Cancer Res Clin Oncol Vol. 124 (1998) pp 565-574) vorgenommen, wobei die Bewertungsergebnisse B(k) zwischen 0 und 1 liegen, und, wenn für das ausgewählte Markerprofil keine fuzzy-basierte Auswerteverfahren bekannt sind, folgendes auf Cut-Off-Werten C(k,m) beruhendes Verfahren angewendet wird:

- wobei für einen Marker m mit dem Cut-Off-Wert C(k,m) die Zugehörigkeitsfunktion Zf(k,m) für die Markerwerte L(m) gebildet wird:

$$Zf(k,m) = \min \left\{ 1; \frac{L(m)}{2 \cdot C(k,m)} \right\}$$

und

daraus die Gesamtbewertung B(k) für das Karzinom k ermittelt wird

$$B(k) = \max_{m=1 \ldots m_M} Zf(k,m) \quad ;$$

| Verwendet man: | | | | |
|---|---|---|---|---|
| C(Lunge, NSE)<br>C(Lunge, CYFRA)<br>C(Brust, CYFRA)<br>C(Brust, CA19-9) | = 13.0<br>= 3,3<br>= 4,5<br>= 20,5 | d.h. | Zf(Lunge, NSE)<br>Zf(Lunge, CYFRA)<br>Zf(Brust, CYFRA)<br>Zf(Brust, CA19-9) | = 0,48<br>= 0,42<br>= 0,30<br>= 0,19 |
| Daraus folgt: | | | B(Lunge) = 0,48<br>B(Brust) = 0,30 | |

8. wenn das Bewertungsergebnis B(k) des Karzinomes k > 0,5 ist, sind die anerkannten diagnostischen Verfahren zu empfehlen;

9. wenn das Bewertungsergebnis B(k) des Karzinomes k < 0,2 ist, ist ein Termin zur Wiederholungsmessung festzulegen, wobei die Wiederholungsmessung mit unverändertem Markerprofil durchzuführen ist;

10. wenn das Bewertungsergebnis B(k) des Karzinomes k > 0,2 ist, die organspezifischen Marker m neu zu bewerten sind, indem die Modifikation ausgeführt wird

$$A^*(k,m) = A(k,m) + \max\{0, B(k) - 0,2\} - A(k,m) \cdot \max\{0, B(k) - 0,2\}$$

wenn die Marker m eine positive Wichtung A*(k,m) > 0,0 bis 1.0 erhalten, die auch für andere als bislang betrachtete organspezifische Karzinome k Bedeutung besitzen, ist mit Schritt 2 fortzufahren; ansonsten ist mit Schritt 5 fortzufahren.

Im Beispielfall ergibt sich zur Anwendung der Schritt 8 bis 11 folgendes:

| Marker Organ | NSE | CEA | CYFRA | CA19-9 | ... |
|---|---|---|---|---|---|
| Lunge | 1 | 0,28 | 0,62 | - | |
| Brust | 0,15 | - | 1 | 0,55 | |
| Haut | - | - | - | - | A(k,m) |

Erhalten hierbei solche Marker eine positive Wichtung, die auch für andere als bislang betrachtete organspezifische Karzinome K Bedeutung besitzen, ist mit Schritt 2 fortzufahren.

Mit den Beispieldaten würde CEA eine positive Wichtung erhalten und es ist zu überprüfen, ob es organspezifische Karzinome gibt, für die CEA ein Marker 1. oder 2. Wahl ist.

Andernfalls ist mit Schritt 5 fortzufahren: entsprechend der geänderten Wichtungen sind Änderungen in der Profilauswahl vorzunehmen. Mit den Beispieldaten findet man nun

W(NSE) = 1,00
W(CEA) = 0,28
W(CYFRA) = 1,00
W(CA19-9) = 0,275

Vorgenannte Verfahrensschritte sind im Ablaufplan gemäß Figur 1 wiedergegeben.

## Patentansprüche

**1.** Gen- und risikoassoziierte Tumormarkerauswahl, **dadurch gekennzeichnet, dass**

1. ein Gen-Test aus dem patienteneigenen Zellgewebe einer Person hinsichtlich krebsrelevanter Gen-Mutationen M(1)..., $M(m_M)$ entsprechend des öffentlichen Wissenstandes durchgeführt wird, deren Ergebnis bezogen auf den gesuchten Mutanten zahlenmäßig wie folgt dargestellt wird: Mutation beobachtet = 1; Mutation

nicht beobachtet = 0;

2. nachfolgend Krebsrisikofaktoren R(1) ... $R(r_R)$, die aus dem öffentlichen Wissensstand bekannt sind, durch Befragung der Personen als karzinomspezifische Einflussfaktoren abgeglichen werden und dem jeweiligen Karzinom zugeordnet werden;

3. die Aussagen aus den Verfahrensschritten 1 und 2 mittels einer tabellarischen Übersicht über den Zusammenhang zwischen organspezifischen Karzinomen und den Gen-Mutanten sowie organspezifischen Karzinomen und den Krebsrisikofaktoren auf ihren potentiellen Risikoanteil zahlenmäßig dargestellt werden, wobei die Tabellenwerte grundsätzlich mit dem Maximalwert 1 zu begrenzen sind;

4. nachfolgend die zahlenmäßige Darstellung des Untersuchungsergebnisses bezüglich der Mutation m mit M(m) und die zahlenmäßige Darstellung des Einflusses des Krebsrisikofaktors r mit R(r) bezeichnet wird, um aus der Gleichung

$$K(k) = 1-\left(\prod_{m=1}^{m_M}(1-M(m)\cdot ZM(m,k))\right)\cdot\left(\prod_{r=1}^{r_R}(1-R(r)\cdot ZR(r,k))\right)$$

den organspezifischen Karzinomverdacht K(k) bezüglich des Karzinoms K zu ermitteln;

5. aus der Fachliteratur organspezifische Marker 1. und 2. Wahl bekannt sind, deren einzelne Wichtungen W (m) in Verbindung mit dem organspezifischen Karzinomverdacht K(k) für jeden einzelnen Marker m aus der Gleichung

$$W(m) = \min\left\{1;\ \sum_{m=1,\,A(k,m)="-"}^{m_W} K(k)\cdot A(k,m)\right\}$$

ermittelt werden, um auf dieser Basis ein Markerprofil für die tumorspezifischen Parameteranalyse auszuwählen, wobei A(k,m) die Eignung des Markers m als Indikator für Karzinome K am Organ einen Zahlenwert zwischen 0 und 1 ausweist;

6. eine Probennahme und Labormessung für eine Markerprofilauswertung nach den bekannten Arbeitsschritten zur Bestimmung der Markerwerte L(m) in Blutserum erfolgt;

7. danach anhand der Markerwerte L(m) Aussagen zur Karzinomdiagnostik mit Hilfe bekannter fuzzy-basierter Auswerteverfahren vorgenommen werden, wobei die Bewertungsergebnisse B(k) zwischen 0 und 1 liegen, und, wenn für das ausgewählte Markerprofil keine fuzzy-basierte Auswerteverfahren bekannt sind, folgendes auf Cut-Off-Werten C(k,m) beruhendes Verfahren angewendet wird:

- wobei für einen Marker m mit dem Cut-Off-Wert C(k,m) die Zugehörigkeitsfunktion Zf(k,m) für die Markerwerte L(m) gebildet wird:

$$Zf(k,m) = \min\left\{1;\frac{L(m)}{2\cdot C(k,m)}\right\}$$

und

- daraus die Gesamtbewertung B(k) für das Karzinom k ermittelt wird

$$B(k) = \max_{m=1 \ldots m_M} Zf(k,m) \quad ;$$

8. wenn das Bewertungsergebnis B(k) des Karzinomes k > 0,5 ist, sind die anerkannten diagnostischen Verfahren zu empfehlen;

9. wenn das Bewertungsergebnis B(k) des Karzinomes k < 0,2 ist, ist ein Termin zur Wiederholungsmessung festzulegen, wobei die Wiederholungsmessung mit unverändertem Markerprofil durchzuführen ist;

10. wenn das Bewertungsergebnis B(k) des Karzinomes k > 0,2 ist, die organspezifischen Marker m neu zu bewerten sind, indem die Modifikation ausgeführt wird

$$A^*(k,m) = A(k,m) + \max\{0; B(k) - 0{,}2\} - A(k,m) \cdot \max\{0; B(k) - 0{,}2\}$$

und

11. wenn die Marker m eine positive Wichtung A*(k,m) > 0,0 bis 1.0 erhalten, die auch für andere als bislang betrachtete organspezifische Karzinome k Bedeutung besitzen, ist mit Schritt 2 fortzufahren; ansonsten ist mit Schritt 5 fortzufahren.

Gen- und risikoassoziierte Tumormarkerauswahl

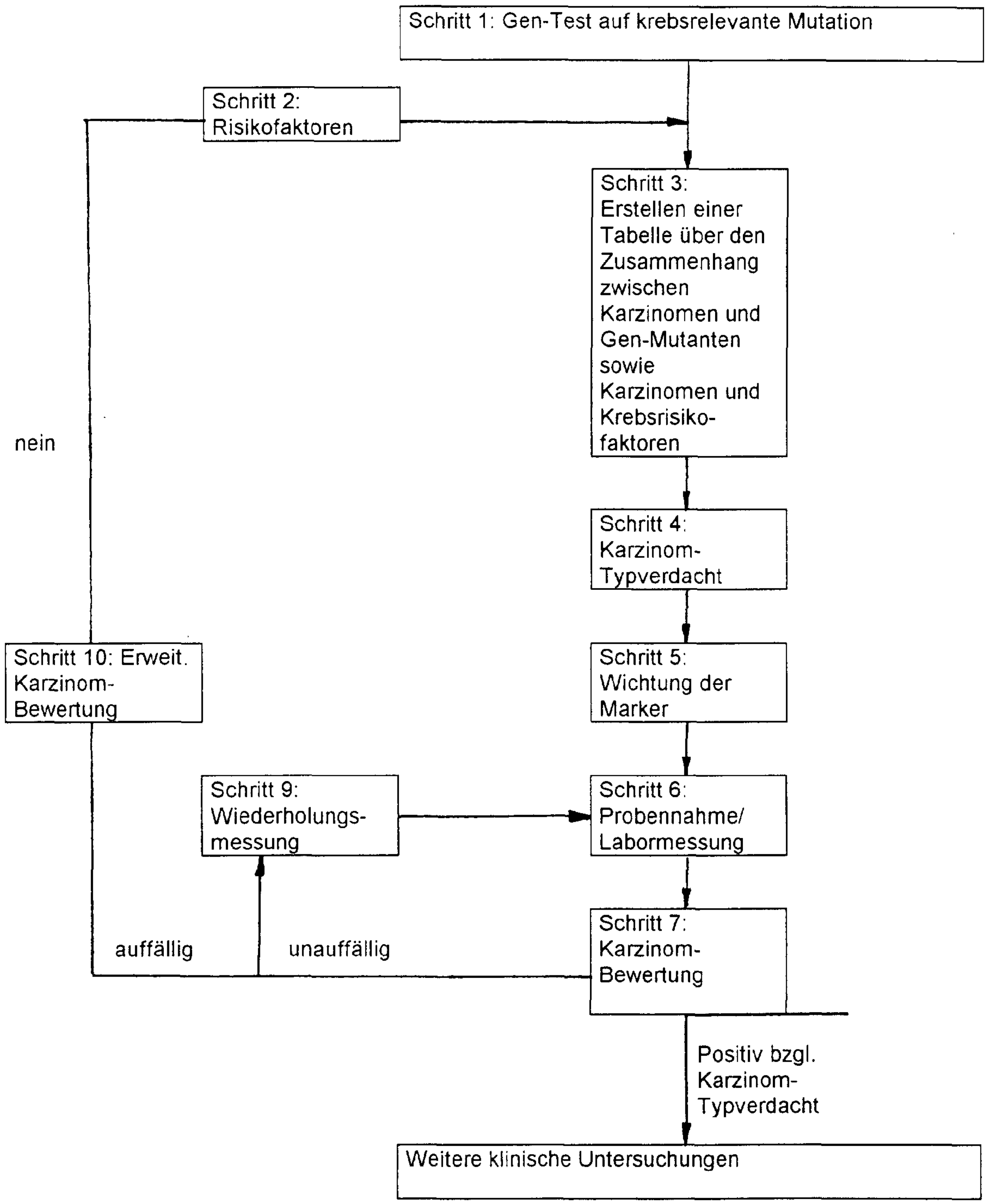


Figur 1